# EUROPÄISCHE PATENTANMELDUNG

(11) **EP 4 112 088 A1**
(43) Veröffentlichungstag der Anmeldung: **04.01.2023**
(21) Anmeldenummer: 21183212.6
(22) Anmeldetag: 01.07.2021
(51) Int. Cl.: A61L 9/14

(54) **VORRICHTUNG UND VERFAHREN ZUM DESINFIZIEREN EINES INNENRAUMS**

(71) Anmelder: Molinari Rail AG, 8400 Winterthur (CH)
(72) Erfinder: WAGNER, Joachim, 8144 Tobelbad (CH); KÜMIN, Daniel, 3122 Kehrsatz (CH); MOLINARI, Michele, 8542 Wiesendangen (CH)
(74) Vertreter: Rutz, Andrea

(57) **Zusammenfassung**

Es wird eine Vorrichtung angegeben zum Desinfizieren eines Innenraums (1), insbesondere eines Innenraums eines Fahrzeugs, aufweisend zumindest eine Zerstäuberdüse (10), zumindest einen Behälter (8) für ein Desinfektionsmittel sowie eine Fördereinrichtung (9, 12, 13). Die Fördereinrichtung (9, 12, 13) dient dazu, das Desinfektionsmittel unter Druck vom zumindest einen Behälter (8) zur zumindest einen Zerstäuberdüse (10) zu befördern und in zerstäubter Form in den Innenraum (1) einzuleiten. Die Vorrichtung weist ausserdem eine elektronische Steuereinheit (11) auf, um das Einleiten des Desinfektionsmittels in den Innenraum (1) zeitlich und/oder bezüglich der Menge automatisch zu steuern. Die Steuereinheit (11) ist zur Desinfektion des Innenraums (1) in mehreren aufeinanderfolgenden Zyklen konfiguriert, wobei jeder Zyklus eine Einbringphase aufweist, während welcher das Desinfektionsmittel in den Innenraum (1) eingeleitet wird, sowie eine darauffolgende Einwirkphase aufweist, während welcher kein Desinfektionsmittel in den Innenraum (1) eingeleitet wird. Ausserdem wird ein Verfahren zum Desinfizieren eines Innenraums angegeben.

## Beschreibung

### TECHNISCHES GEBIET

Die vorliegende Erfindung betrifft eine Vorrichtung sowie ein Verfahren zum Desinfizieren eines Innenraums, insbesondere eines Innenraums eines Fahrzeugs.

### STAND DER TECHNIK

Mobiliar sowie Innenflächen von öffentlich zugänglichen und von stark frequentierten Räumen müssen aus hygienischen Gründen von Zeit zu Zeit desinfiziert werden, um Mikroorganismen sowie Keime wie Bakterien und Viren abzutöten. In Fahrzeugen des öffentlichen Transportsystems, wie insbesondere Schienenfahrzeugen, Bussen, Flugzeugen, Taxis etc., ist das Problem der Keimbildung besonders ausgeprägt, da diese Fahrzeuge über die Dauer von z.B. einem Tag üblicherweise von einer sehr grossen Zahl von Personen benutzt werden.

Eine regelmässige und gründliche Desinfektion ist insbesondere zu Epidemie- und Pandemiezeiten notwendig, wie zum Beispiel während der zum Anmeldezeitpunkt der vorliegenden Anmeldung grassierenden Covid-19 Pandemie. Durch die regelmässige Desinfektion kann verhindert werden, dass Viren auf den Innenflächen sowie dem Mobiliar liegen bleiben oder als Aerosol in der Luft des Innenraums verbleiben. Infektionen und eine Virusausbreitung im öffentlichen Transportsystem können dadurch reduziert werden.

Die Desinfektion durch Reinigungspersonal von Hand ist aufwändig und entsprechend kostspielig. Es besteht dementsprechend ein Bedarf nach automatisierten Lösungen, bei denen die Desinfektion maschinell und mit möglichst wenig Aufwand durchgeführt wird.

Die CN 101850706 A offenbart eine Vorrichtung, bei der ein gasförmiges Oxidans in die Klimaanlage sowie das Fahrzeug eingeleitet wird.

In der DE 10 2013 221 366 A1 wird vorgeschlagen, mittels Sensoren das Niessen einer Person in einem Fahrzeug zu detektieren, um bei erfolgter Detektion eine desinfizierende Substanz in die aus einem Luftauslass strömende Luft einzuleiten und dadurch die Ausbreitung von Krankheitserregern zu verhindern.

Ein weiteres System zum Desinfizieren eines Innenraums mittels Einleiten eines Desinfektionsmittels in die Klimaanlage ist in der CN 106739967 A angegeben.

Aus der US 2011/0195651 A1 ist eine Klimaanlage bekannt, bei der unmittelbar vor dem Verdampfer eine Düse angeordnet ist, um ein Desinfektionsmittel in den Luftstrom einzusprühen und in den Fahrgastraum eines Fahrzeugs einzuleiten. Das Einsprühen wird durch Betätigung eines im Fahrgastraum angeordneten Schalters ausgelöst.

Während die oben erwähnten Dokumente das Desinfizieren der jeweiligen Innenräume betreffen, sind zahlreiche weitere Dokumente bekannt und nachfolgend erwähnt, welche sich auf die Verhinderung der Keimbildung in der Klimaanlage beziehen. Eine Desinfektion des Raumes wird dabei jedoch nicht erreicht.

In der WO 2013/131579 A1 wird eine Vorrichtung offenbart, bei der über eine Sprühvorrichtung ein Behandlungs- oder Desinfektionsmittel in die Klimaanlage eines Fahrzeugs eingesprüht wird. Mit Hilfe des Desinfektionsmittels soll der Verdampfer der Klimaanlage desinfiziert und mit Hilfe des Behandlungsmittels der Fahrzeuginnenraum gereinigt werden.

In der DE 10 2007 057 167 A1 wird eine Fahrzeugklimaanlage offenbart, bei welcher ein Ozon-haltiges Desinfektionsmittel in den durch die Klimaanlage strömenden Luftstrom eingeleitet wird, um die Keimbildung in der Klimaanlage und insbesondere im Verdampfer zu bekämpfen.

Ein weiteres Dokument, welches die Desinfektion der Klimaanlage mittels Einleiten eines Desinfektionsmittel offenbart, betrifft die DE 10 2014 205 018 A1.

In der FR 2 955 031 A1 wird ein kompaktes Desinfektionssystem für einen Bus gezeigt, bei dem das Desinfektionsmittel mit Hilfe von Druckluft durch Zerstäuberdüsen hindurch in den Fahrzeugraum eingeleitet wird.

Die DE 10 2010 047 190 A1 betrifft ein Verfahren zur Desinfektion von Räumen, bei dem im Umluftbetrieb einer Klimaanlage ein Desinfektionsmittel via Vernebelungsdüsen zerstäubt wird. Die Vernebelungsdüsen sind zwischen den Ausströmdüsen für die aus der Klimaanlage in den Innenraum strömende Luft angeordnet.

In der US 2019/0176768 A1 ist ein Desinfektionssystem zur Anordnung unterhalb der Sitze eines Personenfahrzeugs offenbart. Das System weist ein Steuergerät auf, welches über Sensoren z.B. die Öffnung der Fahrzeugtüren detektiert und basierend darauf automatisch eine Pumpe aktiviert, um Desinfektionsmittel aus einem Reservoir in den Fahrzeuginnenraum zu befördern.

Die US 2010/0219259 A1 bezieht sich auf die Luftdesinfektion in Gebäuden. Hierzu wird ein kompaktes System vorgeschlagen, bei dem ein Reservoir und eine Pumpe in einem gemeinsamen Gehäuse angeordnet sind. Mittels der Pumpe wird das Desinfektionsmittel aus dem Reservoir einer Zerstäuberdüse zugeführt, die in der Luftleitung einer Klimaanlage angeordnet ist. Die Steuerung erfolgt via ein Steuergerät.

### DARSTELLUNG DER ERFINDUNG

Es ist eine Aufgabe der vorliegenden Erfindung, eine Vorrichtung anzugeben, mit welcher ein Innenraum effizient und auf möglichst einfache Art und Weise desinfiziert werden kann.

Zur Lösung dieser Aufgabe wird eine Vorrichtung zum Desinfizieren eines Innenraums vorgeschlagen, wie sie in Anspruch 1 angegeben ist. Ausserdem wird in Anspruch 15 ein Verfahren zum Desinfizieren eines Innenraums angegeben. Weitere Ausführungsformen sind in den abhängigen Ansprüchen angegeben.

Die vorliegende Erfindung betrifft also eine Vorrichtung zum Desinfizieren eines Innenraums, insbesondere eines Innenraums eines Fahrzeugs, aufweisend zumindest eine Zerstäuberdüse;
zumindest einen Behälter zur Aufnahme und Lagerung eines Desinfektionsmittels;
eine Fördereinrichtung, um das Desinfektionsmittel unter Druck vom zumindest einen Behälter zur zumindest einen Zerstäuberdüse zu befördern und durch diese hindurch in zerstäubter Form in den Innenraum einzuleiten; sowie
eine elektronische Steuereinheit, um das Einleiten des Desinfektionsmittels in den Innenraum zeitlich und/oder bezüglich der Menge automatisch zu steuern.

Die Steuereinheit ist zur Desinfektion des Innenraums in mehreren aufeinanderfolgenden Zyklen konfiguriert, wobei jeder Zyklus eine Einbringphase aufweist, während welcher das Desinfektionsmittel in den Innenraum eingeleitet wird, sowie eine darauffolgende Einwirkphase aufweist, während welcher kein Desinfektionsmittel in den Innenraum eingeleitet wird.

Das Durchführen der Desinfektion des Innenraums in mehreren derartigen Zyklen hat sich als besonders effizient herausgestellt. Es konnte experimentell nachgewiesen werden, dass auf diese Weise innerhalb verhältnismässig kurzer Zeit eine sehr wirkungsvolle Desinfektion eines Innenraums möglich ist. Das mehrfache aufeinanderfolgende Einbringen des Desinfektionsmittels mit jeweils einer anschliessenden Einwirkphase hat sich als besonders wirkungsvoll zur Inaktivierung von Keimen innerhalb kurzer Zeit in einem Innenraum herausgestellt. Die Einwirkphase ermöglicht es dem Desinfektionsmittel, sich vollständig im Innenraum auszubreiten und auf den Oberflächen abzusetzen. Durch das wiederholte Einbringen werden verbleibende lebende Keime aus dem vorhergehenden Zyklus ebenfalls inaktiviert.

Die Steuereinheit ist bevorzugt dazu konfiguriert oder dazu konfigurierbar, dass die Keimreduktion im Innenraum durch die Desinfektion einer log 4-Stufe, noch bevorzugter gar einer log 5-Stufe, oder besser entspricht. In Bezug auf Desinfektionsvorgänge sind log-Stufen eine dem Fachmann vertraute Angabe zur Beurteilung der Keimreduktion. Dabei entspricht jede log-Stufe jeweils einer Zehnerpotenz. Somit bedeutet eine log 1-Stufe eine Reduktion der Keime um 90 % und eine log 4-Stufe eine Reduktion um 99.99%. Im Falle, dass die Desinfektion einer log 4-Stufe entspricht, werden also beispielsweise 100'000'000 Mikroorganismen auf 10'000 reduziert. Mit einer Desinfektion des Innenraums ist vorteilhaft eine Desinfektion gemeint, die mindestens der log 3-Stufe, bevorzugt aber mindestens der log 4-Stufe, entspricht. In Bezug auf den Innenraum bezieht sich die Angabe der log-Stufe auf sämtliche im Innenraum anwesenden Keime bzw. Mikroorganismen.

Die Steuereinheit kann eine Sicherheitseinrichtung aufweisen, welche dazu ausgebildet ist, die Aktivierung der Desinfektion zu verifizieren und/oder die Durchführung der Zyklen zu überwachen. Alternativ oder zusätzlich kann die Vorrichtung einen oder mehrere Sensoren aufweisen, welche mit der Steuereinheit verbunden sind und dazu dienen, die Desinfektionswirkung und/oder -notwendigkeit zu messen. Die Steuereinheit kann dann insbesondere dazu ausgebildet sein, die Desinfektion in Abhängigkeit der Messwerte des bzw. der Sensoren zu starten und/oder anzupassen, wobei eine Anpassung zum Beispiel bzgl. der Menge an Desinfektionsmittel, der Konzentration an Desinfektionsmittel, der Dauer der Einbringphasen, der Dauer der Einwirkphasen, der Dauer der Belüftungsphase und/oder der Anzahl Zyklen denkbar ist. Die Steuereinheit kann anstelle davon oder zusätzlich aber auch einfach dazu ausgebildet sein, die Desinfektionswirkung und/oder - notwendigkeit z.B. optisch oder akustisch anzuzeigen.

Bei der Desinfektion des Innenraums und somit der Durchführung der erwähnten mehreren aufeinanderfolgenden Zyklen wird der Eintritt in den Innenraum für Personen bevorzugt behindert. Dies kann insbesondere mittels entsprechender Konfiguration der Steuereinheit erreicht werden, welche den Innenraum zum Beispiel für den Eintritt von Personen verschliessen und/oder dies entsprechend, zum Beispiel visuell oder akustisch, signalisieren kann. Dies, weil das Einbringen des Desinfektionsmittels während den Einbringphasen für allfällige im Innenraum anwesende Personen störend oder gar schädlich sein könnte. Vorteilhaft wird der Innenraum zudem auch derart verschlossen, z.B. indem Fenster und Türen geschlossen werden, dass das Desinfektionsmittel während der Desinfektion nicht aus dem Innenraum entweichen kann. Allenfalls kann aber eine Belüftungs- oder Klimaanlage vorhanden sein, die das Desinfektionsmittel z.B. im Umluftbetrieb aus dem Innenraum hinaus und dann wieder in diesen hinein befördert. Die Steuereinheit kann dazu konfiguriert sein, das Verschliessen des Innenraums für Personen und/oder in Bezug auf das Desinfektionsmittel zu Beginn bzw. vor der Desinfektion zu veranlassen und/oder zu überprüfen. Alternativ oder zusätzlich kann die Steuereinheit zudem dazu konfiguriert sein, das Verschliessen des Innenraums im Hinblick auf die Desinfektion zum Beispiel akustisch oder optisch anzuzeigen. Die Steuereinheit kann ausserdem dazu konfiguriert sein, den Innenraum für Personen und/oder in Bezug auf das Desinfektionsmittel zum Schluss bzw. nach der Desinfektion freizugeben, d.h. zum Beispiel Türen und/oder Fenster zu entriegeln oder zu öffnen oder dies dem bzw. den Benutzern mittels eines zum Beispiel optischen oder akustischen Signals anzuzeigen.

Die pro Zyklus jeweils durchgeführte Einbringphase und Einwirkphase folgen bevorzugt jeweils direkt aufeinander, das heisst mit dem Ende der Einbringphase beginnt unmittelbar die Einwirkphase, und, falls es sich nicht um den letzten Zyklus der Desinfektion handelt, beginnt unmittelbar mit dem Ende der Einwirkphase die Einbringphase des nächsten Zyklus'.

Indem eine elektronische Steuereinheit vorhanden ist, kann das Einleiten des Desinfektionsmittels nicht nur zeitlich und bzgl. der Menge optimiert und an die jeweiligen Bedürfnisse angepasst werden, sondern auch derart automatisiert werden, dass die Desinfektion bei Bedarf jeweils weitgehend ohne weitere Benutzereingaben, das heisst vollautomatisch, durchführbar ist.

Die zumindest eine Zerstäuberdüse dient bevorzugt dazu, das in flüssiger Form vorliegende Desinfektionsmittel derart zu zerstäuben, dass sich vorteilhaft ein Aerosol bildet, welches in den Innenraum einströmt und sich in diesem verteilt.

Gemäss einer insbesondere bevorzugten Ausführungsform ist die Steuereinheit zur Desinfektion des Innenraums in genau drei aufeinanderfolgenden Zyklen konfiguriert. Eine Untersuchung mit mehreren durchgeführten Testreihen hat überraschenderweise gezeigt, dass die Wirksamkeit der Desinfektion mit nur zwei Zyklen selbst bei einer Erhöhung der Konzentration des Desinfektionsmittels deutlich reduziert ist. Bei einer Erhöhung der Anzahl Zyklen auf mehr als drei wird hingegen der Verbrauch an Desinfektionsmittel unnötig erhöht.

Des Weiteren konnte in Testreihen festgestellt werden, dass die Einwirkphasen bevorzugt jeweils 3 bis 7 mal, noch bevorzugter 4 bis 5 mal, am bevorzugtesten 4.0 bis 4.5 mal, so lang sind wie die Einbringphasen. Eine Verlängerung der Einbringphasen über diese Relation hinaus führte zu keiner verbesserten Wirkung, sondern lediglich zu einem erhöhten Verbrauch an Desinfektionsmittel.

Insbesondere bevorzugt ist die Steuereinheit ausserdem dazu konfiguriert, nach Durchführen der Zyklen eine Belüftungsphase durchzuführen, um den Innenraum nach Beendigung der Belüftungsphase zum Betreten durch Personen freizugeben. Die Desinfektion des Innenraums beinhaltet dann also die mehreren aufeinanderfolgenden Zyklen mit Einbringphasen und Einwirkphasen sowie im Anschluss an die Zyklen und bevorzugt zum Schluss der Desinfektion eine Belüftungsphase. Die Steuereinheit ist bevorzugt dazu konfiguriert, die Belüftungsphase unmittelbar anschliessend zu den Zyklen durchzuführen, d.h. die Belüftungsphase beginnt unmittelbar mit dem Ende der Einwirkphase des letzten Zyklus'. Während der Belüftungsphase wird die Konzentration an Desinfektionsmittel in der Raumluft vor der erneuten Benutzung des Innenraums reduziert. Die Steuereinheit ist bevorzugt dazu konfiguriert, den Innenraum während der Belüftungsphase mit Frischluft von ausserhalb des Innenraums zu belüften. Hierzu kann die Steuereinheit beispielsweise dazu ausgebildet sein, eines oder mehrere Fenster und/oder Türen zu öffnen und/oder eine Belüftungs- oder Klimaanlage derart anzusteuern, dass von dieser Frischluft in den Innenraum eingebracht wird.

Die erwähnte Belüftungsphase ist bevorzugt 1 bis 3 mal, bevorzugter 1.5 bis 2.5 mal, so lang, wie jeweils eine Einwirkphase. Es hat sich gezeigt, dass eine kürzere Belüftungsphase nicht lange genug ist, um den Innenraum im Hinblick auf die anschliessende Benutzung ausreichend zu belüften. Umgekehrt hatte es sich auch gezeigt, dass eine längere Belüftungsphase nicht notwendig ist, da die Konzentration an Desinfektionsmittel in der Raumluft bereits ausreichend tief ist.

In einer insbesondere bevorzugten Ausführungsform ist die Steuereinheit dazu konfiguriert, die Desinfektion des Innenraums innerhalb von weniger als 240 Minuten, noch bevorzugter sogar innerhalb von weniger als 180 Minuten, durchzuführen. Ein derartiger Zeitrahmen erlaubt es in den meisten Fällen, den Innenraum während einem Zeitraum zu desinfizieren, in welchem der Innenraum sowieso nicht benutzt wird, wie beispielsweise in der Nacht oder, falls es um einen Innenraum eines Fahrzeugs handelt, während eines Stillstands ohne Benutzung von Fahrgästen. Mit den oben erwähnten Verhältnissen der Dauer von Einbringphasen, Einwirkphasen und gegebenenfalls Belüftungsphase ist es mit der hier angegebenen Vorrichtung möglich, den Innenraum innerhalb von weniger als 240 Minuten bzw., bevorzugter, innerhalb von weniger als 180 Minuten, zu desinfizieren, wobei die Desinfektion insbesondere einer log 4-Stufe entsprechen kann.

Bevorzugt weist die Vorrichtung eine Vielzahl derartiger Zerstäuberdüsen auf. Diese Zerstäuberdüsen sind zudem vorteilhaft gleichmässig im Innenraum verteilt. Mit einer gleichmässigen Verteilung im Innenraum ist gemeint, dass die Zerstäuberdüsen in regelmässigen Abständen zueinander derart im Innenraum angeordnet sind, dass sämtliche Bereiche des Innenraumes gleichmässig desinfiziert werden. Das Desinfektionsmittel kann dadurch gleichmässig in den Innenraum eingebracht werden und diesen dementsprechend räumlich gleichmässig und umfassend desinfizieren. Besonders vorteilhaft ist es, wenn die Zerstäuberdüsen im Bereich einer Decke des Innenraums, d.h. in Gravitationsrichtung oben, angeordnet sind. Das Desinfektionsmittel kann dann aufgrund der Schwerkraft langsam nach unten fallen und sich entsprechend im Innenraum verteilen.

Alternativ oder zusätzlich kann zumindest eine Zerstäuberdüse der Vorrichtung zum Anordnen in einer der Belüftung und/oder Klimatisierung des Innenraums dienenden Belüftungs- oder Klimaanlage oder in einer damit verbundenen Luftleitung ausgebildet sein. Das Desinfektionsmittel wird dann während der Einbringphasen durch diese Zerstäuberdüse hindurch in zerstäubter Form in die Belüftungs- oder Klimaanlage oder in die damit verbundene Luftleitung eingeleitet. Durch das Einleiten des Desinfektionsmittels in die Belüftungs- oder Klimaanlage oder in die damit verbundene Luftleitung kann dieses nicht nur zur Desinfektion des Innenraums dienen, sondern auch zur Desinfektion der Belüftungs- oder Klimaanlage und der damit verbundenen Luftleitungen.

Oft weist eine Belüftungsanlage zumindest ein Luftfördermittel, wie insbesondere einen Ventilator oder Verdichter auf. Die Belüftungsanlage kann jedoch z.B. auch lediglich aus einem Rohr- und Klappensystem bestehen, das den bei einem Fahrzeug entstehenden Fahrtwind in den Fahrgastraum leitet. Eine Klimaanlage weist in der Regel ein Heizelement, einen Verdampfer oder beides auf.

Um eine optimale Desinfektion der Belüftungs- oder Klimaanlage zu erreichen, wird die Zerstäuberdüse bevorzugt luftstromaufwärts des Verdampfers angeordnet, falls ein solcher vorhanden ist. Eine optimale Zuführung des Desinfektionsmittels in den Innenraum sowie eine besonders einfache Montage der Zerstäuberdüse ergibt sich aber, wenn die Zerstäuberdüse in einer Zuluftleitung angeordnet wird, die von der Belüftungs- oder Klimaanlage in den Innenraum führt. Alternativ kann die Zerstäuberdüse aber auch in einer Umluftleitung angeordnet sein, welche vom Innenraum zurück in die Belüftungs- oder Klimaanlage führt.

Indem die Zerstäuberdüse zum Anordnen in einer der Belüftung und/oder Klimatisierung des Innenraums dienenden Belüftungs- oder Klimaanlage oder in einer damit verbundenen Luftleitung ausgebildet ist, wird die zur Belüftung bzw. Klimatisierung sowieso vorhandene Infrastruktur benutzt, um das Desinfektionsmittel vorteilhaft als Aerosol im Innenraum zu verteilen.

Der Innenraum ist bevorzugt nach aussen hin weitgehend abschliessbar, so dass das eingeleitete Aerosol im Innenraum verbleibt und nur zu sehr geringen oder gar vernachlässigbaren Anteilen nach aussen gelangen kann.

Beim Innenraum kann es sich insbesondere um den Innenraum eines Fahrzeugs handeln. Das Fahrzeug dient bevorzugt als öffentliches Transportmittel. Es kann zum Beispiel ein Schienenfahrzeug, ein Bus oder ein Flugzeug sein. Möglich ist aber auch, dass es sich beim Innenraum um den Raum eines Gebäudes handelt. Das Gebäude kann zum Beispiel ein öffentlich zugängliches Gebäude, ein Einkaufszentrum, ein Shop, ein Hotel oder ein Restaurant sein.

Der zumindest eine Behälter ist bevorzugt ein nach aussen hin weitgehend abschliessbarer Behälter, so dass das darin gelagerte Desinfektionsmittel nicht nach aussen hin entweichen kann. Je nachdem welches Desinfektionsmittel verwendet wird, kann dieses zum Beispiel in konzentrierter oder in gelöster Form im Behälter gelagert werden.

Die elektronische Steuereinheit weist bevorzugt ein programmierbares Bauteil sowie ein Speicherelement auf. Vorteilhaft weist es auch eine Verarbeitungseinheit, wie insbesondere einen Mikroprozessor auf. Das Speicherelement kann insbesondere eine Leiterplatte umfassen. Mittels des programmierbaren Bauteils und des Speicherelements ist das Einleiten des Desinfektionsmittels bevorzugt hinsichtlich des Zeitverlaufs und der Menge einstellbar.

Bevorzugt weist die Vorrichtung ausserdem eine Bedieneinheit zum Bedienen und Einstellen der Steuereinheit auf. Die Bedieneinheit kann unmittelbar in der Nähe der Steuereinheit angeordnet sein. Sie kann sogar zusammen mit der Steuereinheit auf einer gemeinsamen Leiterplatte angebracht sein. Bevorzugt ist die Bedieneinheit jedoch zur beabstandeten Anordnung bzgl. der Steuereinheit ausgebildet. Auf diese Weise kann die Steuereinheit zum Beispiel zusammen mit der Zerstäuberdüse, dem Behälter und der Fördereinrichtung im Inneren des Innenraums und/der im unmittelbaren Bereich der Belüftungs- oder Klimaanlage oder in der damit verbundenen Luftleitung angeordnet werden, und die Bedieneinheit kann entfernt davon, insbesondere ausserhalb des Innenraums, an einer für den Benutzer gut zugänglichen Stelle platziert werden. Die Kommunikation zwischen der Steuereinheit und der Bedieneinheit kann kabelgebunden oder drahtlos sein.

Die Bedieneinheit kann zur Anordnung innerhalb des Innenraums ausgebildet sein. Eine sich im Innenraum aufhaltende Person kann so direkt vor Ort die Steuereinheit programmieren, um zum Beispiel die Zeitintervalle der Desinfektion und/oder die Menge an Desinfektionsmittel einzustellen. In anderen, ebenfalls bevorzugten Ausführungsformen kann die Bedieneinheit aber auch zur Anordnung ausserhalb des Innenraums ausgebildet sein. Die bedienende Person kann sich dadurch, wenn eine Desinfektion des Innenraums vorgenommen werden soll, aus dem Innenraum entfernen, diesen verschliessen und den Desinfektionsprozess starten. Ein Kontakt von Menschen mit einer grossen Menge an Desinfektionsmittel kann dadurch vermieden werden. Ein Betreten des Innenraums kann nach erfolgter Desinfektion wieder ermöglicht werden, sobald sich das Desinfektionsmittel verflüchtigt hat.

In einer insbesondere bevorzugten Ausführungsform ist die Bedieneinheit und/oder die Steuereinheit ausserdem zur Ansteuerung der Belüftungs- oder Klimaanlage ausgebildet. Mittels entsprechender Ansteuerung durch die Bedien- oder Steuereinheit kann die Belüftungs- oder Klimaanlage während des Desinfektionsvorgangs derart betrieben werden, dass das Desinfektions-Aerosol optimal im Innenraum verteilt wird. Beispielsweise kann die Förderung der durch die Belüftungs- oder Klimaanlage strömenden Luft auf ein gewünschtes Mass erhöht oder verringert werden und/oder die Belüftungs- oder Klimaanlage kann in einen Umluftbetrieb geschaltet werden, so dass kein Desinfektionsmittel aus dem Innenraum nach draussen gelangt. Indem der Betrieb der Belüftungs- oder Klimaanlage auf den Desinfektionsvorgang abgestimmt wird, kann eine besonders effiziente Desinfektion des Innenraumes erreicht werden.

In der Steuereinheit sind bevorzugt mehrere Betriebsmodi vorkonfiguriert. Die Desinfektionsvorrichtung kann dadurch bedarfsgemäss eingesetzt und betrieben werden.

Vorteilhaft ist in der Steuereinheit zumindest ein Modus vorkonfiguriert, bei dem das Desinfektionsmittel bei entsprechender Bedienung der Bedieneinheit unmittelbar derart in die Belüftungs- oder Klimaanlage oder die damit verbundene Luftleitung eingeleitet wird, dass eine vollständige Desinfektion des Innenraums durchgeführt wird. Mit einer vollständigen bzw. kompletten Desinfektion des Innenraums ist bevorzugt eine Desinfektion gemeint, welche bezüglich ihrer Wirksamkeit zumindest der log 4-Stufe entspricht. Auf eine entsprechende Eingabe an der Bedieneinheit hin wird also unmittelbar eine vollständige Desinfektion des Innenraums durchgeführt. Mit "unmittelbar" ist gemeint, dass die Desinfektion zeitlich unverzüglich oder mit einer kurzen Zeitverzögerung durchgeführt wird. Eine kurze Zeitverzögerung kann zum Beispiel dazu dienen, dass sich im Innenraum noch anwesende Personen entfernen können.

Alternativ oder zusätzlich kann in der Steuereinheit zumindest ein Modus vorkonfiguriert sein, bei dem das Desinfektionsmittel in regelmässigen Abständen in den Innenraum eingeleitet wird. Auf diese Weise kann einer Keimbildung zum Beispiel auch während der laufenden Benutzung des Innenraumes mittels Einleiten einer verhältnismässig kleinen Menge an Desinfektionsmittel entgegengewirkt werden. Im Gegensatz dazu kann eine vollständige Desinfektion zum Beispiel jeweils abends durchgeführt werden, wenn sich keine Personen mehr im Innenraum aufhalten, wobei dann eine verhältnismässig grosse Menge an Desinfektionsmittel zum Einsatz kommen kann.

Alternativ oder zusätzlich kann in der Steuereinheit zumindest ein Modus vorkonfiguriert sein, der eine Einstellung bezüglich der zeitlichen und/oder mengenmässigen Einleitung des Desinfektionsmittels durch den Benutzer erlaubt. Dieser Modus erlaubt eine optimale Anpassung des Desinfektionsvorgangs an die jeweiligen Gegebenheiten des Innenraums und der Belüftungs- oder Klimaanlage durch den Benutzer.

Bei der Fördereinrichtung kann es sich zum Beispiel um eine Elektropumpe handeln, welche das Desinfektionsmittel aus dem Behälter zur Zerstäuberdüse transportiert. Alternativ kann die Fördereinrichtung auch die Druckbeaufschlagung des Behälters mit einem Gas umfassen, so dass das Desinfektionsmittel aufgrund des Gasdrucks aus dem Behälter hinaus und zur Zerstäuberdüse getrieben wird. In einer insbesondere bevorzugten Ausführungsform weist die Fördereinrichtung jedoch eine Druckluftquelle oder einen Anschluss an eine Druckluftquelle auf, welche über eine Leitung mit dem Behälter verbunden ist, so dass das Desinfektionsmittel mittels Druckluft aus dem Behälter zur Zerstäuberdüse förderbar ist. Die Verwendung einer Druckluftquelle hat den Vorteil, dass sich das Desinfektionsmittel bereits zum Teil mit der Luft vermischt und ein Aerosol bildet, bevor es durch die Zerstäuberdüse gelangt. Ausserdem ist ein Anschluss an eine Druckluftquelle gerade bei Schienenfahrzeugen meist bereits vorhanden.

Eine besonders einfache Steuerung der Desinfektion wird ermöglicht, wenn in der Leitung zwischen der Druckluftquelle bzw. dem Anschluss an die Druckluftquelle und dem Behälter ein Stellventil angeordnet ist, welches von der Steuereinheit ansteuerbar ist, um den Durchfluss von Druckluft von der Druckluftquelle zum Behälter zu steuern.

Das Desinfektionsmittel enthält bevorzugt Wasserstoffperoxid (H₂O₂), wobei das Wasserstoffperoxid insbesondere bevorzugt mit einer Konzentration von 5 bis 20 %, bevorzugter von 5 bis 15% und noch bevorzugter mit einer Konzentration von 5 bis 10 % im Desinfektionsmittel vorliegt. Es hat sich gezeigt, dass eine derartige Konzentration zu optimalen Ergebnissen führt. Die Desinfektionswirkung basiert somit bevorzugt hauptsächlich auf der Oxidation von Wasserstoffperoxid. Der Vorteil von Wasserstoffperoxid besteht darin, dass dieses in Wasser- und Sauerstoffmoleküle zerfällt und dadurch für die Umwelt und für Personen, welche den Innenraum nach dem Desinfektionsvorgang benutzen, unbedenklich ist. Selbstverständlich können aber auch andere Substanzen als Desinfektionsmittel verwendet werden. So kann das Desinfektionsmittel zum Beispiel auch auf der Wirkung von Chlordioxid, Ozon, Alkohol, Chlor, Natriumhypochlorit oder einer beliebigen anderen bekannten Substanz sowie auf Kombinationen davon basieren.

In einer insbesondere bevorzugten Ausführungsform hat die Vorrichtung eine insgesamt kompakte Bauweise und ist zum Nachrüsten eines bestehenden Innenraums, insbesondere eines Innenraums seines Fahrzeugs, und/oder einer bestehenden Belüftungs- oder Klimaanlage ausgebildet. Mit einer kompakten Bauweise ist gemeint, dass die Fördereinrichtung, die Steuereinheit und der Behälter für das Desinfektionsmittel unmittelbar in der Nähe voneinander angeordnet sind und dadurch auf einfache Art und Weise mittels entsprechender Anordnung der Zerstäuberdüse(n), beispielweise durch Anschluss an eine bestehende Belüftungs- oder Klimaanlage, zur Desinfektion eines Innenraums eingesetzt werden können. Vorteilhaft bildet die Vorrichtung mit der Fördereinrichtung, der Steuereinheit und dem Behälter für das Desinfektionsmittel eine weitgehend eigenständige funktionelle Komponente, insbesondere auch dann, wenn die Vorrichtung in Kombination mit einer Belüftungs- oder Klimaanlage zum Einsatz kommt. Die Fördereinrichtung, die Steuereinheit und der Behälter für das Desinfektionsmittel können insbesondere in oder an einem gemeinsamen Gehäuse angeordnet sein.

Die Erfindung betrifft ausserdem ein Verfahren zum Desinfizieren eines Innenraums, bevorzugt eines Innenraums eines Fahrzeugs, insbesondere mittels einer Vorrichtung wie oben angegeben, das Verfahren aufweisend zumindest die folgenden Schritte:
- Befördern eines Desinfektionsmittels unter Druck zu zumindest einer Zerstäuberdüse mittels einer Fördereinrichtung, um das Desinfektionsmittel durch die zumindest eine Zerstäuberdüse hindurch in zerstäubter Form in den Innenraum einzuleiten, sowie
- Steuern des Einleitens des Desinfektionsmittels in den Innenraum zeitlich und/oder bezüglich der Menge.

Das Steuern des Einleitens erfolgt dabei automatisch mittels einer elektronischen Steuereinheit derart, dass die Desinfektion des Innenraums in mehreren aufeinanderfolgenden Zyklen durchgeführt wird, wobei jeder Zyklus eine Einbringphase aufweist, während welcher das Desinfektionsmittel in den Innenraum eingeleitet wird, sowie eine darauffolgende Einwirkphase aufweist, während welcher kein Desinfektionsmittel in den Innenraum eingeleitet wird.

Der Innenraum kann zur Belüftung und/oder Klimatisierung eine Belüftungs- oder Klimaanlage aufweisen. Das Desinfektionsmittel kann in diesem Fall in die Belüftungs- oder Klimaanlage oder in eine damit verbundene Luftleitung eingeleitet werden.

Bevorzugt wird nicht nur das Einleiten des Desinfektionsmittels in den Innenraum gesteuert, sondern, falls vorhanden, eine Belüftungs- oder Klimaanlage gleichzeitig derart gesteuert, dass ihr Betrieb auf die Desinfektionsmitteleinleitung abgestimmt ist, insbesondere was die Förderung der durch sie hindurchströmenden Luft angeht.

Das Steuern des Einleitens des Desinfektionsmittels kann insbesondere anhand eines vorgegebenen zeitlichen Verlaufs der Menge der Desinfektionsmitteleinleitung vorgenommen werden. Ein solcher zeitlicher Verlauf ist bevorzugt in der Steuereinheit vorprogrammiert oder vorprogrammierbar.

Bevorzugt wird die Belüftungs- oder Klimaanlage, falls vorhanden, während des Einleitens des Desinfektionsmittels in einen Umluftbetrieb geschaltet, so dass kein oder nur wenig Desinfektionsmittel aus dem Innenraum hinaus nach aussen gelangen kann.

### KURZE BESCHREIBUNG DER ZEICHNUNGEN

Eine bevorzugte Ausführungsform der Erfindung wird im Folgenden anhand einer Zeichnung beschrieben, die lediglich zur Erläuterung dient und nicht einschränkend auszulegen ist. In den Zeichnungen zeigen:
- Fig. 1: eine schematische Ansicht einer Vorrichtung zum Desinfizieren eines Innenraums gemäss einer ersten erfindungsgemässen Ausführungsform;
- Fig. 2: eine schematische Ansicht einer Vorrichtung zum Desinfizieren eines Innenraums gemäss einer zweiten erfindungsgemässen Ausführungsform;
- Fig. 3: eine Darstellung der Positionierung der Messstellen in einem Eisenbahnwagen bei einem konkret durchgeführten Experiment unter Benutzung einer erfindungsgemässen Vorrichtung;
- Fig. 4a: eine schematische Darstellung der verschiedenen Phasen des in einem ersten Versuch durchgeführten Desinfektionsverfahrens;
- Fig. 4b: die beim ersten Versuch gemäss Fig. 4a gemessenen Kurvenverläufe;
- Fig. 5a: eine schematische Darstellung der verschiedenen Phasen des in einem zweiten Versuch durchgeführten Desinfektionsverfahrens;
- Fig. 5b: die beim zweiten Versuch gemäss Fig. 5a gemessenen Kurvenverläufe;
- Fig. 6a: eine schematische Darstellung der verschiedenen Phasen des in einem dritten Versuch durchgeführten Desinfektionsverfahrens;
- Fig. 6b: die beim dritten Versuch gemäss Fig. 6a gemessenen Kurvenverläufe;
- Fig. 7a: eine schematische Darstellung der verschiedenen Phasen des in einem vierten Versuch durchgeführten Desinfektionsverfahrens; sowie
- Fig. 7b: die beim vierten Versuch gemäss Fig. 7a gemessenen Kurvenverläufe.

### BESCHREIBUNG BEVORZUGTER AUSFÜHRUNGSFORMEN

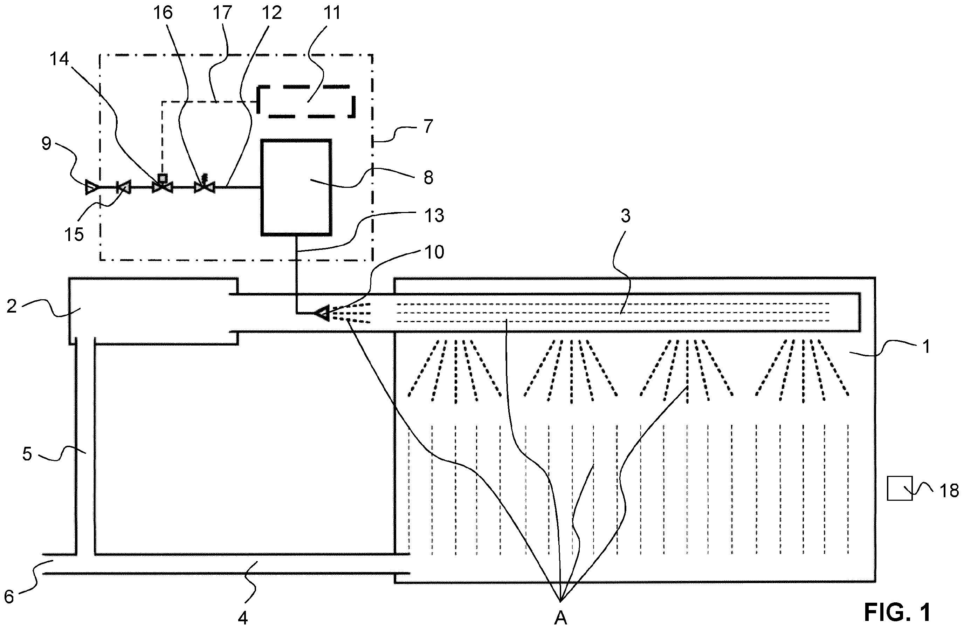

In der Figur 1 ist schematisch eine bevorzugte Ausführungsform der Erfindung dargestellt. Dabei wird hier eine modulartige Vorrichtung verwendet, um einen Innenraum 1 mit einer bestehenden, das heisst sowieso vorhandenen Belüftungs- oder Klimaanlage 2 zu desinfizieren und somit die Ausbreitung und Festsetzung von z.B. Viren, Bakterien und anderen Keimen zu verhindern. Personen, welche sich im Innenraum 1 befinden, sollen dadurch wirksam vor einer Infektion mit den Keimen geschützt werden.

Beim Innenraum 1 kann es sich um den Raum eines Gebäudes handeln. Insbesondere bevorzugt handelt es sich jedoch um den Fahrgastraum eines Fahrzeugs, wie insbesondere eines Schienenfahrzeugs oder eines Kraftfahrzeugs, also zum Beispiel eines Busses oder eines Automobils. Der Innenraum 1 ist vorteilhaft zumindest für die Dauer des Desinfektionsvorgangs nach aussen hin im Wesentlichen abschliessbar, so dass kein oder nur vernachlässigbar wenig Desinfektionsmittel-Aerosol nach aussen gelangen kann.

Bei diesem Ausführungsbeispiel ist zur Belüftung und/oder Klimatisierung des Innenraums 1 eine Belüftungs- oder Klimaanlage 2 vorgesehen. Falls es sich um eine Belüftungsanlage handelt, weist diese vorteilhaft einen Ventilator oder einen Verdichter auf, um Aussenluft in den Innenraum 1 zu befördern. Falls eine Klimaanlage vorgesehen ist, weist diese bevorzugt zumindest ein Heizelement und/oder einen Verdampfer auf, um die in den Innenraum 1 eingeleitete Luft zu temperieren.

Im vorliegenden Ausführungsbeispiel gelangt der Luftstrom nach der Belüftungs- oder Klimaanlage 2 via eine Zuluftleitung 3 in den Innenraum 1. Durch eine Abluftleitung 4 hindurch gelangt die Luft wieder aus dem Innenraum 1 hinaus und durch einen Auslass 6 hindurch nach aussen. Die Belüftungs- oder Klimaanlage 2 kann zudem, zum Beispiel mittels in der Zeichnung nicht dargestellten Klappen, in einen Umluftbetrieb geschaltet werden, in dem die aus dem Innenraum 1 ausströmende Luft via eine Umluftleitung 5 wieder in die Belüftungs- oder Klimaanlage 2 gelangt. Ein Teil der Abluftleitung 4 bildet dann ebenfalls einen Teil der Umluftleitung.

Um ein einfache, aber doch sehr effiziente Desinfektion des Innenraums 1 zu ermöglichen, ist modulartig eine erfindungsgemässe Vorrichtung vorgesehen, welche zumindest einen Behälter 8 für Desinfektionsmittel, einen Druckluftanschluss 9, eine Zerstäuberdüse 10 sowie eine Steuereinheit 11 aufweist. Die Vorrichtung zum Desinfizieren des Innenraums 1 bildet somit als Ganzes ein Modul 7, das einfach an die Belüftungs- oder Klimaanlage 2 bzw. die Zuluftleitung 3 anschliessbar ist, um unter Benutzung der bestehenden Belüftungs- oder Klimaanlage 2 eine effiziente Desinfektion des Innenraums 1 zu bewirken.

Das Modul 7 hat bevorzugt als Ganzes eine kompakte Bauweise. Die strichpunktierte Linie in der Figur 1, welche das Modul 7 umrandet, kann in einer insbesondere bevorzugten Ausführungsform auch für ein Gehäuse stehen, in welchem der Behälter 8 und die Steuereinheit 7 sowie weitere Komponenten der erfindungsgemässen Vorrichtung angeordnet sind.

Je nachdem welches Desinfektionsmittel verwendet wird, kann dieses zum Beispiel in konzentrierter oder gelöster Form im Behälter 8 aufgenommen sein. Die Aufbewahrungsform sollte vorteilhaft auf das verwendete Desinfektionsmittel abgestimmt sein. Bevorzugt liegt das Desinfektionsmittel im Behälter 8 in flüssiger Form vor. Der Behälter 8 ist nach aussen hin abschliessbar, so dass via eine daran angeschlossene Druckluftleitung 12 Druckluft in den Behälter 8 eingeleitet werden kann, um das Desinfektionsmittel via eine Rohrleitung 13 zu einer Zerstäuberdüse 10 zu befördern.

Der Druckluftanschluss 9 dient zum Anschliessen der Vorrichtung an eine Druckluftquelle, um Druckluft via die Druckluftleitung 12 zum Behälter 8 zu leiten. Insbesondere bei Schienenfahrzeugen ist eine Druckluftquelle oft bereits vorhanden. Der Druckluftanschluss 9, die Druckluftleitung 12 und die Rohrleitung 13 bilden somit gemeinsam eine Fördereinrichtung, um das Desinfektionsmittel unter Druck vom Behälter 8 zur Zerstäuberdüse 10 zu befördern.

Die Zerstäuberdüse 10 ist im Inneren der Zuluftleitung 3 angeordnet. Die Rohrleitung 13 erstreckt sich entsprechend durch die Wandung der Zuluftleitung hindurch. Falls die erfindungsgemässe Vorrichtung bei diesem Ausführungsbeispiel nachgerüstet werden soll, muss somit einfach ein Zugang für die Zerstäuberdüse 10 in die Zuluftleitung 3 (oder in die Belüftungs- oder Klimaanlage 2 oder in die Abluftleitung 4 oder in die Umluftleitung 5) geschaffen werden. Dies kann zum Beispiel mittels einer Bohrung sowie allenfalls anschliessender Abdichtung problemlos bewerkstelligt werden. Aufgrund der kompakten Bauweise des Moduls 7 ist dieses für ein Nachrüsten einer bestehenden Belüftungs- oder Klimatisierungssystems besonders gut geeignet.

Das Desinfektionsmittel wird von der Zerstäuberdüse 10 zerstäubt und gelangt somit via diese in das Innere der Zuluftleitung 3. Durch das Zerstäuben bildet das Desinfektionsmittel mit der durch die Belüftungs- oder Klimaanlage 2 strömenden Luft unmittelbar ein Aerosol A, welches via entsprechende Einlassöffnungen in den Innenraum 1 gelangt und sich in diesem verteilt.

Im Innenraum 1 setzt sich das Aerosol A auf den Oberflächen ab und desinfiziert diese dadurch. Vorteilhaft ist, wenn die Belüftungs- oder Klimaanlage 2 während des Desinfektionsvorgangs in einen Umluftbetrieb geschaltet wird, so dass das Aerosol A via die Umluftleitung 4, 5 wieder in die Belüftungs- oder Klimaanlage 2 und von dieser erneut in den Innenraum 1 gelangt, ohne sich in die Umgebung zu verflüchtigen. Auf diese Weise wird zudem nicht nur der Innenraum 1 desinfiziert, sondern auch die Belüftungs- oder Klimaanlage 2 sowie die Leitungen 3, 4 und 5.

Um ein Rückströmen der Druckluft zu verhindern, ist innerhalb der Druckluftleitung 12 ein Rückschlagventil 15 angeordnet. Ausserdem ist ein Anschlussventil 16 vorhanden, welches einen einfachen Austausch des Behälters 8 ermöglicht, zum Beispiel wenn dieser leer ist.

Zwischen dem Rückschlagventil 15 und dem Anschlussventil 16 ist ein Stellventil 16 angeordnet, welches zur Regelung des Druckluftdurchflusses durch die Druckluftleitung 12 hindurch dient. Indem der Druckluftdurchfluss durch die Druckluftleitung 12 geregelt wird, kann auch das Einleiten von Desinfektionsmittel in die Zuluftleitung 3 gesteuert werden. Die Steuerung des Stellventils 16 erfolgt mittels der Steuereinheit 11, welche via eine Steuerverbindung 17 mit dem Stellventil 16 verbunden ist.

Bei der Steuereinheit 11 handelt es sich um eine elektronische Steuereinheit, mittels welcher das Einleiten des Desinfektionsmittels in die Zuluftleitung 3 zeitlich und/oder bezüglich der Menge automatisch steuerbar ist. In der Steuereinheit 11 können insbesondere zeitliche Verläufe gespeichert oder vorprogrammiert sein, gemäss welchen das Desinfektionsmittel in die Zuluftleitung eingeleitet wird. Beispielsweise kann vorgesehen sein, dass eine Desinfektion des Innenraums 1 jeweils nach Betriebsschluss, das heisst zum Beispiel abends, durchgeführt wird, wenn sich keine Personen mehr im Innenraum 1 aufhalten. Alternativ oder zusätzlich kann auch vorgesehen sein, dass eine geringe Menge an Desinfektionsmittel kontinuierlich oder in regelmässigen Abständen während des laufenden Betriebs in den Innenraum 1 eingeleitet wird, um eine Virenausbreitung bereits dann zu verhindern.

Die Steuereinheit 11 ist insbesondere dazu konfiguriert, die Desinfektion des Innenraums 1 in mehreren, vorzugsweise genau drei, aufeinanderfolgenden Zyklen durchzuführen, wobei jeder Zyklus eine Einbringphase aufweist, während welcher das Desinfektionsmittel via die Zerstäuberdüse 10 in den Innenraum 1 eingeleitet wird, sowie eine darauffolgende Einwirkphase aufweist, während welcher das Einleiten von Desinfektionsmittel in den Innenraum 1 unterbrochen ist. Ausserdem ist die Steuereinheit 11 bevorzugt dazu konfiguriert, nach Durchführung dieser Zyklen den Innenraum 1 zu belüften. Dies kann beispielsweise mittels Umschaltung der Belüftungs- oder Klimaanlage 2 derart erreicht werden, dass von dieser Frischluft in den Innenraum 1 eingeleitet wird. Zusätzlich oder alternativ kann die Steuereinheit 11 zum Beispiel auch ein visuelles oder akustisches Signal abgeben, um die Durchführung der Belüftungsphase anzuzeigen und den bzw. die Benutzer entsprechend zum Öffnen von Fenstern und/oder Türen aufzufordern. Die Steuereinheit 11 ist zudem bevorzugt dazu konfiguriert, Personen am Eintritt in den Innenraum 11 für den Zeitraum der Desinfektion zu behindern, was beispielsweise mittels Verschliessen aller Zugangstüren und/oder mittels entsprechender zum Beispiel visueller oder akustischer Signalisation erreicht werden kann.

Zum Modul 7 kann eine Bedieneinheit 18 gehören, mittels welcher die Steuereinheit zum Beispiel ein- und ausgeschaltet, programmiert und eingestellt werden kann. Die Bedieneinheit 18 kann innerhalb oder ausserhalb des Innenraumes 1 an einer für den Benutzer gut zugänglichen Stelle angeordnet sein. Um eine komplette Desinfektion des Innenraums 1 mit einer verhältnismässig grossen Menge an Desinfektionsmittel zu ermöglichen, ist die Bedieneinheit im vorliegenden Ausführungsbeispiel ausserhalb des Innenraums 1 angeordnet. Der Bedienende kann dadurch vorgängig sicherstellen, dass sich keine Personen mehr im Innenraum 1 befinden, den Innenraum 1 verlassen und dann den Desinfektionsvorgang von ausserhalb des Innenraums 1 mittels entsprechender Eingabe an der Bedieneinheit 18 auslösen. Selbstverständlich kann die Desinfektion auch automatisch, beispielsweise mit Hilfe einer Zeitschaltuhr und/oder aufgrund von Sensormessungen, ausgelöst werden.

Zur Desinfektion des Innenraums 1 wird also das Stellventil 14 von der Steuereinheit 11 automatisch so verstellt, dass vom Druckluftanschluss 9 Druckluft in den Behälter 8 eingeleitet wird und das darin enthaltene Desinfektionsmittel unter Druck zur Zerstäuberdüse 10 befördert. Von der Zerstäuberdüse 10 wird das Desinfektionsmittel zerstäubt, wodurch es mit der die Belüftungs- oder Klimaanlage 2 durchströmenden Luft ein Aerosol A bildet. Das Aerosol A gelangt via die Zuluftleitung 3 in den Innenraum 1, wo es sich verteilt und auf den Oberflächen absetzt. Allfällig vorhandene Mikroorganismen und Keime werden dadurch abgetötet. Die Belüftungs- oder Klimaanlage 2 befindet sich dabei vorteilhaft im Umluftbetrieb, so dass das Aerosol A via die Umluftleitung 4, 5 wieder in die Belüftungs- oder Klimaanlage 2 und von dieser erneut in den Innenraum 1 gelangt. Dabei werden auch die Belüftungs- oder Klimaanlage 2 sowie die Leitungen 3, 4, 5 und insbesondere ein allfällig in der Belüftungs- oder Klimaanlage 2 vorhandener Verdichter desinfiziert. Falls es sich um eine vollständige Desinfektion des Innenraums 1 handelt, für welche alle Personen den Innenraum zuvor verlassen müssen, kann nach erfolgter Desinfektion das Stellventil 14 mittels der Steuereinheit 11 verschlossen und der Innenraum 1 kann nach einer gewissen, der Belüftung dienenden Wartezeit wieder zum Betreten freigegeben werden.

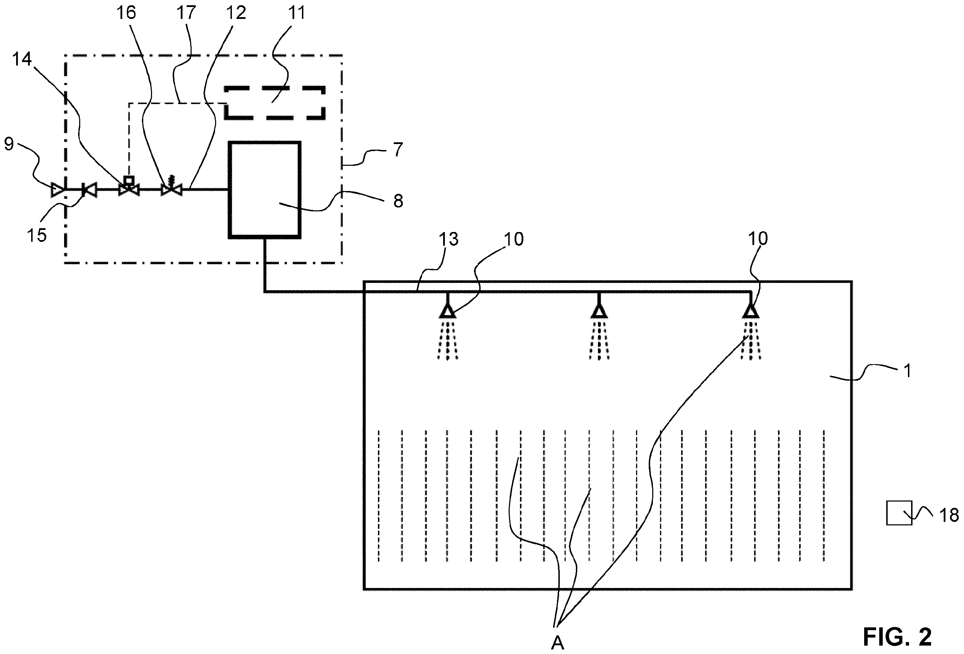

In der Figur 2 ist eine weitere Vorrichtung zum Desinfizieren eines Innenraums 1 gemäss einer zweiten erfindungsgemässen Ausführungsform gezeigt. Die Vorrichtung wird nachfolgend anhand der Unterschiede zu der in der Figur 1 gezeigten Ausführungsform beschrieben.

Das Desinfektionsmittel wird hier nicht in eine Belüftungs- oder Klimaanlage 2 bzw. in eine damit verbundene Luftleitung eingeleitet, sondern direkt in den Innenraum 1. Es sind hierzu mehrere Zerstäuberdüsen 10 vorgesehen, welche gleichmässig verteilt an der Decke des Innenraums 1 angeordnet sind. Die Rohrleitung 13 verteilt das Desinfektionsmittel zu den verschiedenen Zerstäuberdüsen 10. Von den Zerstäuberdüsen 10 aus breitet sich das Aerosol A mit dem Desinfektionsmittel gleichmässig im Innenraum 1 aus und setzt sich aufgrund der Schwerkraft auf den Oberflächen des Innenraums 1 ab. Auch wenn in der Figur 2 keine gezeigt ist, kann eine Belüftungs- oder Klimaanlage selbstverständlich auch hier vorhanden sein. Das Modul 7 ist im Wesentlichen identisch wie dasjenige der Figur 1 ausgebildet.

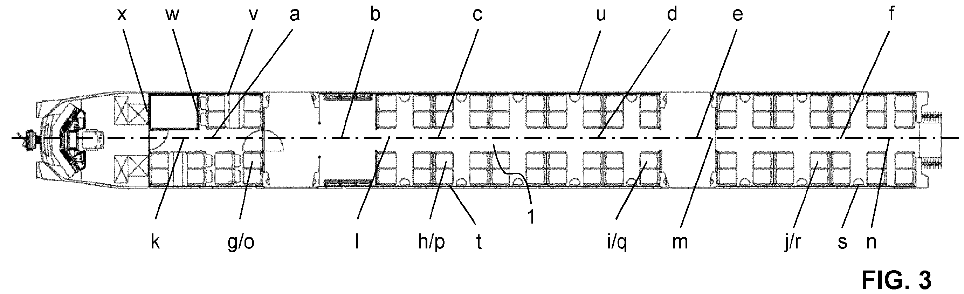

In der Figur 3 ist im Grundriss ein Eisenbahnwagen gezeigt, der eine erfindungsgemässe Vorrichtung zum Desinfizieren des Innenraums 1 aufweist. Zum Kontrollieren und Verifizieren der Desinfektionswirkung sind im Innenraum 1 verteilt eine Vielzahl von Messstellen a-x angeordnet. Die Messstellen a-x dienen jeweils zum Messen der Wirkung des Desinfektionsmittels an der entsprechenden Stelle mittels chemischer und biologischer Indikatoren, wobei für den Nachweis der Desinfektionswirkung hauptsächlich die biologischen Indikatoren entscheidend sind. Um ein aussagekräftiges und umfassendes Messresultat zu erhalten, sind 6 Messstellen (a-f) jeweils an der Mitte des Deckenkanals angeordnet, 4 Messstellen (g-j) jeweils an der Unterseite eines Fahrgastsitzes angrenzend zum Mittelgang, 4 Messstellen (k-n) jeweils mittig im Mittelgang am Boden, 4 Messstellen (o-r) jeweils an der Oberseite eines Fahrgastsitzes angrenzend zum Mittelgang, 4 Messstellen (s-v) jeweils oberhalb und mittig eines Fensters, sowie 2 Messstellen (w, x) im unmittelbaren Bereich des WC's (an der Deckenkante gegenüber des Lüfters sowie am Boden unter dem Lüfter). Die Wirkung des Desinfektionsmittels im Innenraum 1 kann dadurch für verschiedenste Bereiche des Eisenbahnwagens optimal kontrolliert werden.

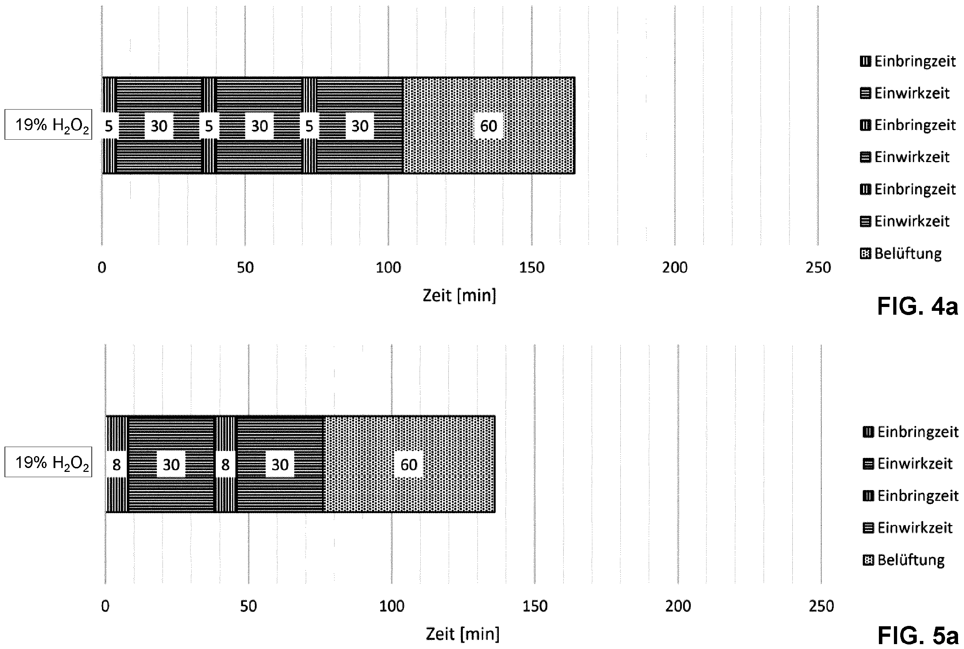

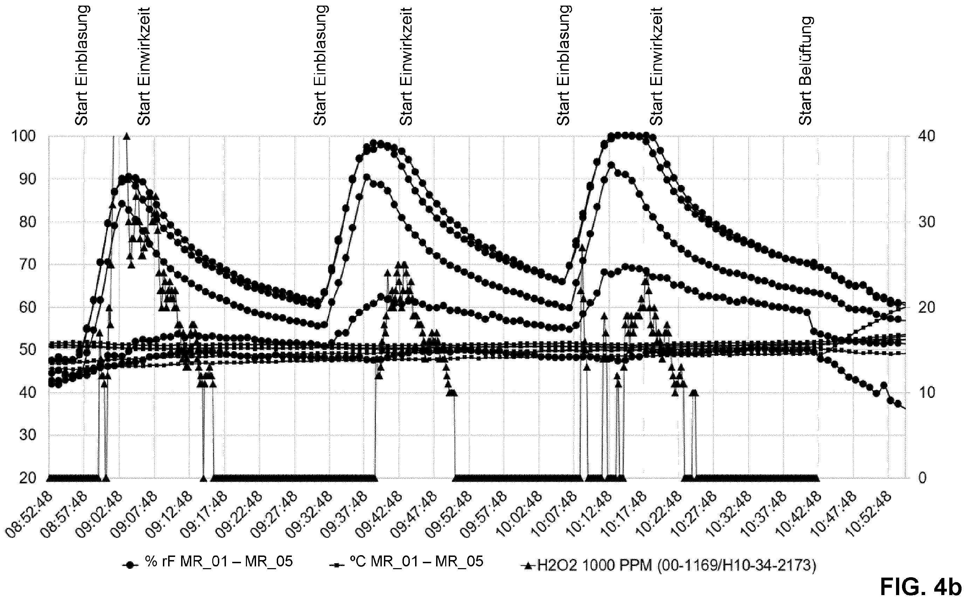

Die Figuren 4a, 5a, 6a und 7a zeigen verschiedene Versuche eines Desinfektionsverfahren, welches bei einem konkreten Experiment in jeweils demselben Innenraum eines Eisenbahnwagens durchgeführt wurden. Es sind das jeweils verwendete Desinfektionsmittel sowie die im Zeitverlauf nacheinander durchgeführten Phasen inkl. Minutenangaben angegeben. Die Figuren 4b, 5b, 6b und 7b zeigen die jeweils dazugehörigen Messresultate, wobei die horizontale Achse jeweils die Zeitachse bildet, die primäre (links beschriftete) Vertikalachse die relative Luftfeuchtigkeit (rF) in % bzw. die H₂O₂-Konzentration in PPM angibt, und die sekundäre (rechts beschriftete) Vertikalachse die Raumtemperatur in °C anzeigt. Die relative Luftfeuchtigkeit und die Raumtemperatur wird jeweils für verschiedene Messstellen MR_01 - MR_05 aufgeführt, welche 5 der 6 in der Figur 3 gezeigten Messstellen a-f an der Mitte des Deckenkanals entsprechen. Die H₂O₂-Konzentration steht für die relative Menge an Wasserstoffperoxid im gesamten Innenraum 1. Der Beginn der verschiedenen Phasen der Desinfektion ist jeweils angegeben, wobei "Start Einblasung" jeweils den Beginn einer Einbringphase, "Start Einwirkzeit" den Beginn einer Einwirkphase und "Start Belüftung" den Beginn der Belüftungsphase kennzeichnet.

In der nachfolgenden Tabelle 1 sind für jeden der in den Figuren 4a - 7b gezeigten Versuche jeweils der Verbrauch und die Konzentration des Desinfektionsmittels sowie die mittels Messungen bestätige chemische und biologische Wirkung für Populationen von unterschiedlichen log-Stufen angegeben.

**Tabelle 1: Verbrauch an Desinfektionsmittel sowie bestätigte chemische und biologische Wirkung für die verschiedenen, in den Figuren 4a - 7b abgebildeten Versuche.**

| Versuch | Verbrauch an Desinfektionsmittel | Konzentration | Wirkung chemischer Indikatoren | Wirkung biologischer Indikatoren | | |
|---|---|---|---|---|---|---|
| | | | | log 4 Population | log 5 Population | log 6 Population |
| Figuren 4a,b | 1856 g | 19% | 92 % | 88 % | 88 % | 56 % |
| Figuren 5a,b | 1933 g | 19% | 88 % | 88 % | 84 % | 76 % |
| Figuren 6a,b | 2648 g | 7.5% | 92 % | 100 % | 96 % | 44 % |
| Figuren 7a,b | 3689 g | 7.5% | 92 % | 96 % | 96 % | 84 % |

In Vorversuchen konnte bereits festgestellt werden, dass eine Desinfektion, bei welcher das Desinfektionsmittel in mehreren aufeinanderfolgenden Zyklen mit jeweils einer Einbringphase und einer Einwirkphase in den Innenraum 1 eingeleitet wird, besonders effizient bezüglich Zeitdauer und Wirkung ist.

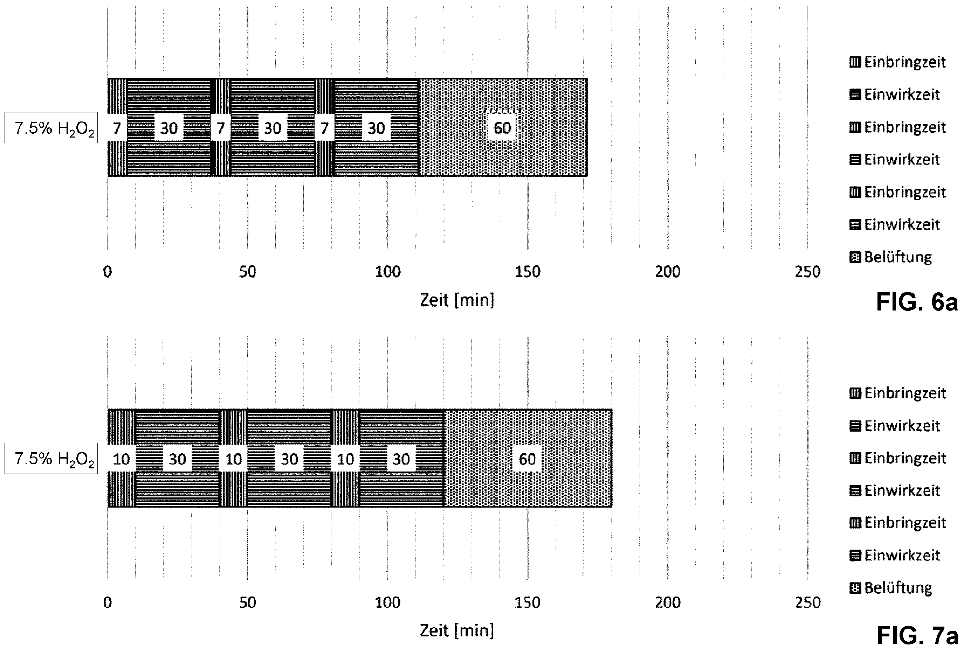

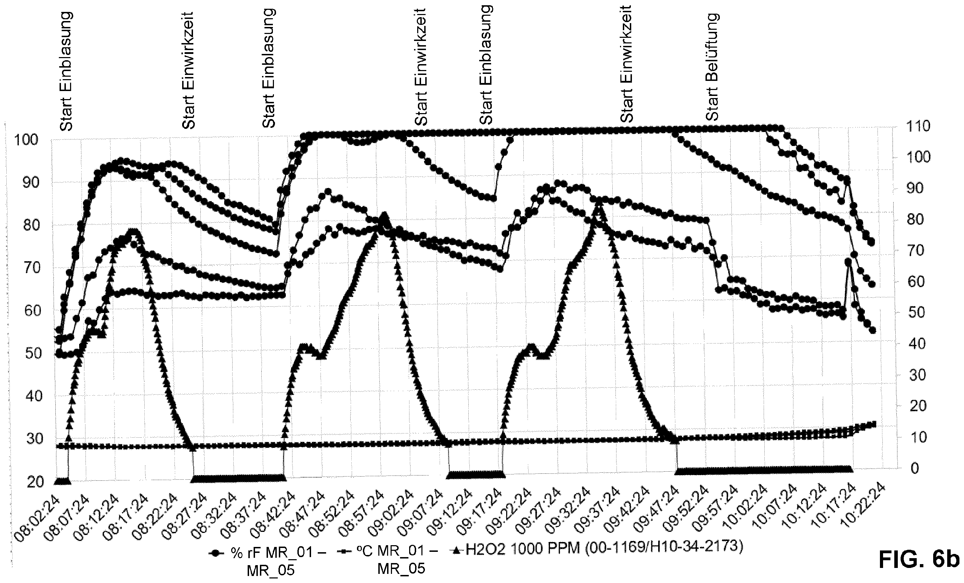

Die weiteren, in den Figuren 4a - 7b dargestellten Versuche zielten insbesondere darauf ab, die biologische Wirkung der Desinfektion in Bezug auf eine log 4-Population noch weiter zu optimieren. Überraschenderweise konnte dabei beim Versuch gemäss den Figuren 6a und 6b die beste Wirkung erzielt werden. Bei diesem Versuch wurde das Einbringen des Desinfektionsmittels in den Innenraum 1 in drei sich wiederholenden Zyklen jeweils während 7 Minuten durchgeführt (Einbringphase) und während 30 Minuten unterbrochen (Einwirkphase). Im Anschluss an die drei Zyklen wurde der Innenraum 1 während 60 Minuten belüftet (Belüftungsphase). Dabei wurde ein Desinfektionsmittel mit einer 7.5%-Konzentration an Wasserstoffperoxid benutzt, wovon eine Menge von insgesamt 2648 g in den Innenraum 1 eingeleitet wurde. Wie aus der Tabelle 1 hervorgeht, konnte bei diesem Versuch als einzigem eine bestätigte 100-prozentige biologische Wirkung in Bezug auf die log 4-Population festgestellt werden. Auch bezüglich der log 5-Population konnte eine immer noch 96-prozentige Wirkung sowie bezüglich der chemischen Indikatoren eine 92-prozentige Wirkung festgestellt werden, was über die vier durchgeführten Versuche hinweg Bestwerte bedeuten.

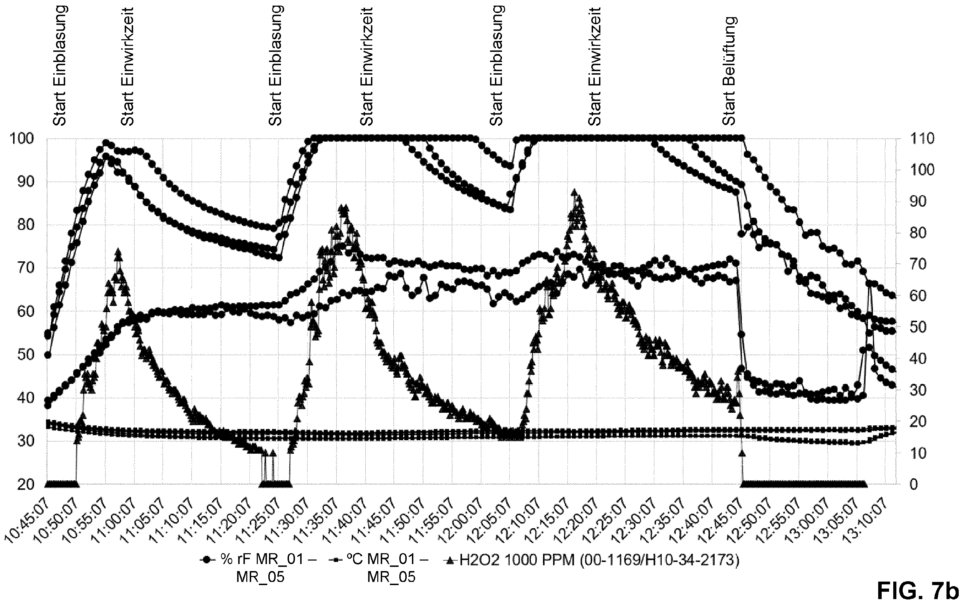

Im Vergleich zu dem in den Figuren 6a und 6b gezeigten Versuch ergab sich beim Versuch der Figuren 7a, 7b, bei dem die Einbringphasen verlängert wurden, keine Verbesserung der Desinfektionswirkung, sondern lediglich einen höheren Verbrauch an Desinfektionsmittel.

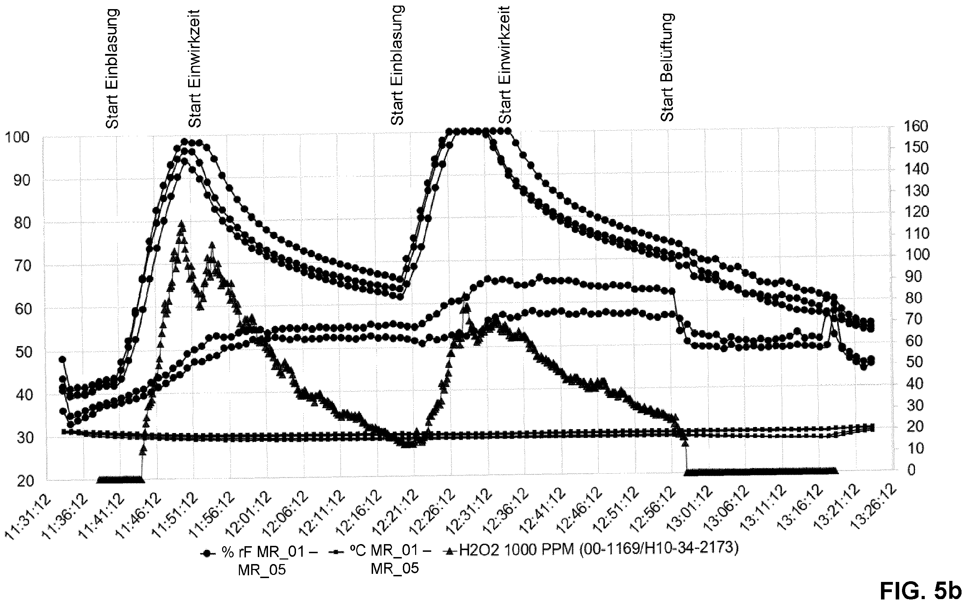

Eine Reduktion der Einbring- und Einwirkphasen um einen Zyklus inklusive Erhöhung der Konzentration des Desinfektionsmittels auf eine 19-prozentige Lösung beim Versuch der Figuren 5a, 5b führte zu einer verminderten Desinfektionswirkung.

Ein bezüglich des Versuchs der Figuren 6a, 6b vergleichbar Desinfektionszyklus inklusive Erhöhung der Konzentration des Desinfektionsmittels auf eine 19-prozentige Lösung beim Versuch der Figuren 4a, 4b führte ebenfalls zu einer verminderten Desinfektionswirkung.

Bei allen durchgeführten Versuchen war die Konzentration an Wasserstoffperoxid in der Raumluft nach Abschluss der Belüftungsphase derart, dass ein gefahrlos Betreten des Innenraumes 1 möglich war. Der Nachweis der biologischen Desinfektionswirkung erfolgte mit biologischen Indikatoren, welche dem industriellen Standard entsprechen und garantieren, dass auch behüllte Viren, wie z.B. SARS-COV-2, inaktiviert werden.

**BEZUGSZEICHENLISTE**

| | | | |
|---|---|---|---|
| 1 | Innenraum | 11 | Steuereinheit |
| | | 12 | Druckluftleitung |
| 2 | Belüftungs-/Klimaanlage | 13 | Rohrleitung |
| 3 | Zuluftleitung | 14 | Stellventil |
| 4 | Abluftleitung | 15 | Rückschlagventil |
| 5 | Umluftleitung | 16 | Anschlussventil |
| 6 | Auslass | 17 | Steuerverbindung |
| 7 | Modul | 18 | Bedieneinheit |
| 8 | Behälter | | |
| 9 | Druckluftanschluss | A | Aerosol |
| 10 | Zerstäuberdüse | a-x | Messstellen |

## Patentansprüche

1. Vorrichtung zum Desinfizieren eines Innenraums (1), insbesondere eines Innenraums eines Fahrzeugs, aufweisend
zumindest eine Zerstäuberdüse (10);
zumindest einen Behälter (8) zur Aufnahme und Lagerung eines Desinfektionsmittels;
eine Fördereinrichtung (9, 12, 13), um das Desinfektionsmittel unter Druck vom zumindest einen Behälter (8) zur zumindest einen Zerstäuberdüse (10) zu befördern und durch diese hindurch in zerstäubter Form in den Innenraum (1) einzuleiten; sowie
eine elektronische Steuereinheit (11), um das Einleiten des Desinfektionsmittels in den Innenraum (1) zeitlich und/oder bezüglich der Menge automatisch zu steuern,
**dadurch gekennzeichnet, dass**
die Steuereinheit (11) zur Desinfektion des Innenraums (1) in mehreren aufeinanderfolgenden Zyklen konfiguriert ist, wobei jeder Zyklus eine Einbringphase aufweist, während welcher das Desinfektionsmittel in den Innenraum (1) eingeleitet wird, sowie eine darauffolgende Einwirkphase aufweist, während welcher kein Desinfektionsmittel in den Innenraum (1) eingeleitet wird.

2. Vorrichtung nach Anspruch 1, wobei die Steuereinheit (11) zur Desinfektion des Innenraums (1) in genau drei Zyklen konfiguriert ist.

3. Vorrichtung nach Anspruch 1 oder 2, wobei die Einwirkphasen jeweils 3 - 7 mal, bevorzugt 4 - 5 mal, so lang sind, wie die Einbringphasen.

4. Vorrichtung nach einem der vorhergehenden Ansprüche, wobei die Steuereinheit (11) ausserdem dazu konfiguriert ist, nach Durchführen der Zyklen eine Belüftungsphase durchzuführen, um den Innenraum (1) nach Beendigung der Belüftungsphase zum Betreten durch Personen freizugeben.

5. Vorrichtung nach Anspruch 4, wobei die Belüftungsphase 1 - 3 mal, bevorzugt 1.5 - 2.5 mal, so lang ist, wie jeweils eine Einwirkphase.

6. Vorrichtung nach einem der vorhergehenden Ansprüche, wobei die Steuereinheit (11) dazu konfiguriert ist, die Desinfektion des Innenraums (1) innerhalb von weniger als 240 Minuten, bevorzugt innerhalb von weniger als 180 Minuten, durchzuführen.

7. Vorrichtung nach einem der vorhergehenden Ansprüche, aufweisend eine Vielzahl derartiger Zerstäuberdüsen (10), welche gleichmässig im Innenraum (1) verteilt und bevorzugt im Bereich einer Decke des Innenraums angeordnet sind.

8. Vorrichtung nach einem der Ansprüche 1 bis 6, wobei die Zerstäuberdüse (10) zum Anordnen in einer der Belüftung und/oder Klimatisierung des Innenraums (1) dienenden Belüftungs- oder Klimaanlage (2) oder in einer damit verbundenen Luftleitung (3) ausgebildet ist.

9. Vorrichtung nach einem der vorhergehenden Ansprüche, ausserdem aufweisend eine Bedieneinheit (18) zum Bedienen und Einstellen der Steuereinheit (11), und wobei die Bedieneinheit (18) bevorzugtzur Anordnung ausserhalb des Innenraums (1) ausgebildet ist.

10. Vorrichtung nach Anspruch 9, wobei die Bedieneinheit (18) und/oder die Steuereinheit (11) ausserdem zur Ansteuerung einer Belüftungs- oder Klimaanlage (2) ausgebildet ist.

11. Vorrichtung nach einem der vorhergehenden Ansprüche, wobei in der Steuereinheit (11) zumindest ein Modus vorkonfiguriert ist, der eine Einstellung bezüglich der zeitlichen und/oder mengenmässigen Einleitung des Desinfektionsmittels durch den Benutzer erlaubt.

12. Vorrichtung nach einem der vorhergehenden Ansprüche, wobei die Fördereinrichtung (9, 12, 13) eine Druckluftquelle oder einen Anschluss an eine Druckluftquelle (9) aufweist, welche über eine Leitung (12) mit dem Behälter (8) verbunden ist, so dass das Desinfektionsmittel mittels Druckluft aus dem Behälter (8) zur Zerstäuberdüse (10) förderbar ist, und wobei in der Leitung (12) zwischen der Druckluftquelle bzw. dem Anschluss an die Druckluftquelle (9) und dem Behälter (8) bevorzugt ein Stellventil (14) angeordnet ist, welches von der Steuereinheit (11) ansteuerbar ist, um den Durchfluss von Druckluft von der Druckluftquelle zum Behälter (8) zu steuern.

13. Vorrichtung nach einem der vorhergehenden Ansprüche, wobei das Desinfektionsmittel Wasserstoffperoxid mit einer Konzentration von 5 - 20 %, insbesondere von 5 - 10 %, enthält.

14. Vorrichtung nach einem der vorhergehenden Ansprüche, wobei die Vorrichtung eine insgesamt kompakte Bauweise hat und zum Nachrüsten eines bestehenden Innenraums (1), insbesondere eines Innenraums eines Fahrzeugs, ausgebildet ist.

15. Verfahren zum Desinfizieren eines Innenraums (1), bevorzugt eines Innenraums eines Fahrzeugs, insbesondere mittels einer Vorrichtung nach einem der vorhergehenden Ansprüche, das Verfahren aufweisend zumindest die folgenden Schritte:
- Befördern eines Desinfektionsmittels unter Druck zu zumindest einer Zerstäuberdüse (10) mittels einer Fördereinrichtung (9, 12, 13), um das Desinfektionsmittel durch die zumindest eine Zerstäuberdüse (10) hindurch in zerstäubter Form in den Innenraum (1) einzuleiten, sowie
- Steuern des Einleitens des Desinfektionsmittels in den Innenraum (1) zeitlich und/oder bezüglich der Menge,
**dadurch gekennzeichnet, dass**
das Steuern des Einleitens automatisch mittels einer elektronischen Steuereinheit (11) derart erfolgt, dass die Desinfektion des Innenraums (1) in mehreren aufeinanderfolgenden Zyklen durchgeführt wird, wobei jeder Zyklus eine Einbringphase aufweist, während welcher das Desinfektionsmittel in den Innenraum (1) eingeleitet wird, sowie eine darauffolgende Einwirkphase aufweist, während welcher kein Desinfektionsmittel in den Innenraum (1) eingeleitet wird.

16. Verfahren nach Anspruch 16, wobei während des Einleitens des Desinfektionsmittels eine der Belüftung und/oder Klimatisierung des Innenraums (1) dienende Belüftungs- oder Klimaanlage (2) in einen Umluftbetrieb geschaltet wird.
